Europäisches Patentamt

European Patent Office

Office européen des brevets

(19)

(11) Veröffentlichungsnummer: **0 291 747**
**A1**

## (12) EUROPÄISCHE PATENTANMELDUNG

(21) Anmeldenummer: 88106882.9

(22) Anmeldetag: 29.04.88

(51) Int. Cl.⁴ **A61K 7/16**

(30) Priorität: 04.05.87 AT 1113/87

(43) Veröffentlichungstag der Anmeldung:
23.11.88 Patentblatt 88/47

(84) Benannte Vertragsstaaten:
AT BE CH DE ES FR GB GR IT LI LU NL SE

(71) Anmelder: **Blendax GmbH**
**Rheinallee 88**
**D-6500 Mainz(DE)**

(72) Erfinder: **Wagner, Helmar R.**
**Römerstrasse 62**
**D-6100 Darmstadt-Arheilgen(DE)**

(54) **Zahnpasta.**

(57) Die vorliegende Erfindung betrifft eine Zahnpasta, die eine die Zahnsteinbildung reduzierende und das Zahnfleisch pflegende Wirkung aufweist.

Diese Zahnpasta ist dadurch gekennzeichnet, daß sie eine Kombination aus

a) einem Di- und/oder Tetraalkalipyrophosphat in einer Menge von 1 bis 5 Gew.% (berechnet auf Pyrophosphat), bezogen auf die Gesamtzusammensetzung, und/oder Ethan-1-hydroxy-1, 1-diphosphonsäure bzw. deren wasserlöslichen Salzen in einer Menge von 0,2 bis 2,0 Gew.% (berechnet auf freie Phosphonsäure), bezogen auf die Gesamtzusammensetzung
und

b) einem wasserlöslichen Salz des 3-Pyridylmethanols in einer Menge von 0,05 bis 0,5% (berechnet auf 3-Pyridylmethanol), bezogen auf die Gesamtzusammensetzung, enthält.

EP 0 291 747 A1

## ZAHNPASTA

Die vorliegende Erfindung betrifft eine Zahnpasta, die eine die Zahnsteinbildung reduzierende und das Zahnfleisch pflegende Wirkung aufweist.

Es sind bereits zahlreiche Wirkstoffe zum Einsatz in Zahn- und Mundpflegemitteln vorgeschlagen worden, die eine die Bildung von Zahnstein verringernde Wirksamkeit aufweisen sollen.

Eine in diesem Zusammenhang bereits umfangreich untersuchte Substanz ist die Ethan-1-hydroxy-1,1-diphosphonsäure (EHDP), die zur Verwendung als zahnsteinhemmendes Mittel erstmals in der FR-A 1 514 194 vorgeschlagen wurde.

Von diesem Vorschlag wird zwischenzeitlich auch kommerziell Gebrauch gemacht, da eine diesen Wirkstoff enthaltende Zahnpasta seit längerem auf dem Markt angeboten wird.

Eine weitere Gruppe von Substanzen, die sowohl in vitro als auch in vivo eine die Zahnsteinbildung vermindernde Wirkung aufweisen, sind Tetraalkalipyrophosphate, insbesondere Tetranatrium- und Tetrakaliumpyrophosphat, vgl. W.W. Briner, M.D. Francis, Calc. Tiss. Res. 11 (1973), 10-22.

Von der Erkenntnis dieser wissenschaftlichen Veröffentlichung macht auch die EP-A 97 476 Gebrauch, die Zahn- und Mundpflegemittel zum Gegenstand hat, die, in an sich üblicher Grundlage (selbstverständlich in Abwesenheit von Calciumionen abspaltenden Verbindungen) Alkali- bzw. Tetraalkalipyrophosphate enthalten.

Derartige Zahnpasten haben in klinischen Untersuchungen eine durchschnittliche Reduktion der Zahnsteinbildung zwischen etwa 26% und etwa 35% erzielt (vgl. J. Amer. Dental Assc. 110 (1985), 737-738, und J. Dental Res. 64 (1985), 1159-1162).

Es wurde nun gefunden, daß sich eine Verbesserung der zahnfleischpflegenden Wirksamkeit einer die Zahnsteinbildung verringernden Zahnpasta mit einem Gehalt an Ethan-1-hydroxy-1,1-diphosphonsäure und/oder Di- bzw. Tetraalkalipyrophosphaten erzielen läßt, wenn man dieser ein Salz des 3-Pyridylmethanols zusetzt.

3-Pyridylmethanol, auch unter der Bezeichnung Beta-Pyridylcarbinol bekannt, weist eine durchblutungsfördernde Wirkung auf und wird in Form seiner Salze, insbesondere des Hydrogentartrats, auch als tonisierender Wirkstoff und als zahnfleischpflegendes Agens in Zahn- und Mundpflegemitteln eingesetzt.

Es war überraschend und nicht vorhersehbar, daß sich durch die an sich ungewöhnliche Kombination dieser Substanzen eine Wirkungssteigerung im Hinblick auf eine verbesserte Zahnfleischpflege erzielen läßt, da die Anwesenheit von Zahnstein nach der geltenden zahnmedizinischen Auffassung in keinem Zusammenhang mit dem Auftreten von Zahnfleischproblemen steht.

Es bedurfte daher einer echten erfinderischen Auswahl, unter den zahlreichen zur Verfügung stehenden Möglichkeiten gerade die eng begrenzten Substanzgruppen auszuwählen, durch deren gemeinsame Anwendung eine überraschende Wirkungssteigerung in an sich unerwarteter Richtung auftritt, ohne die erwarteten Kompatibilitätsprobleme hervorzurufen.

Der Anteil an Ethan-1-hydroxy-1,1-diphosphonsäure in der erfindungsgemäßen Zahnpasta liegt vorzugsweise bei etwa 0,2 bis 2,0 Gew.% der Gesamtzusammensetzung, insbesondere zwischen etwa 0,5 und 1,0 Gew.%.

Soweit die Salze, beispielsweise das Di- und Trinatriumsalz von EHDP, eingesetzt werden, bezieht sich die Berechnung jeweils auf den Anteil an freier Säure.

Geeignete Alkalipyrophosphate sind insbesondere Dinatrium- und Dikalium- sowie Tetranatrium- und Tetrakaliumpyrophosphat bzw. deren Gemische.

Der Anteil dieser Verbindungen in der erfindungsgemäßen Zahnpasta liegt zwischen etwa 1 und etwa 5 Gew.%, vorzugsweise zwischen 2 und 4, insbesondere bei etwa 2,5 bis 3,5 Gew.%, bezogen auf die Gesamtzusammensetzung der Zahnpasta, jeweils berechnet auf das Pyrophosphation.

Gemäß einer bevorzugten Ausführungsform können auch Gemische aus EHDP und Pyrophosphat(en) eingesetzt werden.

Das 3-Pyridylmethanol wird vorzugsweise in Form seiner wasserlöslichen Salze eingesetzt.

Besonders geeignet sind hierbei die Salze mehrbasischer Carbonsäuren, insbesondere das (Hydrogen-)Tartrat, aber auch das Citrat, Gluconat, Lactat, Aspartat, etc., können eingesetzt werden.

Der Anteil an 3-Pyridylmethanolsalz liegt bei 0,05 bis 0,5 Gew.% der Gesamtzusammensetzung, berechnet auf die freie Base.

Die erfindungsgemäßen Zahnpasten enthalten keine Poliermittel, die in der Lage sind, Calciumionen in nennenswertem Umfang abzugeben.

Geeignete Poliermittel sind deshalb insbesondere verschiedene Siliciumdioxid-Modifikationen wie gefällte Silikagele, Siliciumdioxid-Xerogele und -Hydrogele, Alkalialuminiumsilikate, beispielsweise solche vom Zeolith-Typ wie synthetisches Natriumaluminiumsilikat der empirischen Formel $Na_{12}(AlO_2)_{12}(SiO_2)_{12}.27H_2O$, Aluminiumoxid und Aluminiumoxidtrihydrat, unlösliches Metaphosphat, pulverförmige Kunststoffe sowie auch hitzebehandeltes Calciumpyrophosphat, das praktisch keine Calciumionen freisetzt.

Es können selbstverständlich auch Poliermittelgemische aus den genannten Substanzen eingesetzt werden, beispielsweise ein Gemisch aus α-Aluminiumoxidtrihydrat und/oder unlöslichem Alkalimetaphosphat und synthetischem Zeolith A im Verhältnis von etwa 1:1.

Der Poliermittelanteil in den erfindungsgemäßen Zahnpasten liegt vorzugsweise zwischen etwa 20 und 60 Gew.% der Gesamtzusammensetzung.

Es ist selbstverständlich möglich, die üblichen in Zahnpasten eingesetzten oberflächenaktiven Verbindungen in Mengen bis zu etwa 2,5 Gew.-% der Gesamtzusammensetzung zu verwenden.

Solche synthetischen oberflächenaktiven Stoffe sind beispielsweise Alkylsulfate, Alkylethersulfate, Olefinsulfonate, Natriumlauroylsarcosinat oder ampholytische, nichtionische oder kationaktive Verbindungen oder auch Seifen wie beispielsweise solche von Laurinsäure, Myristinsäure, Palmitinsäure, Stearinsäure oder Gemischen derselben, beispielsweise Cocosölfettsäuren oder Talgfettsäuren.

Gleiches gilt hinsichtlich der in Zahnpasten üblicherweise in Mengen zwischen etwa 10 und etwa 35 Gew.-% Einsatz gelangenden Feuchthaltemittel, wie Glycerin, Diolen wie 1,4-Butandiol oder 1,2-Propandiol oder Zuckeralkoholen wie Sorbit, Mannit oder Xylit und Polyglykolen mit niederen Molekulargewichten, ebenso für Verdickungsmittel, deren Mengenanteil in Zahnpasten zwischen etwa 0,25 und etwa 2,5 Gew.-% der Gesamtzusammensetzung liegt.

Bevorzugte Verdickungsmittel sind Carboxymethylcellulose und deren Alkalisalze, insbesondere Natriumcarboxymethylcellulose, Hydroxyalkylcellulosen wie Hydroxymethylcellulose und Hydroxyethylcellulose, Methycellulose, Pflanzengummen wie Tragant, Gummi arabicum, Carayagummi, Guargummi, Xanthangummi und Irish Moos, synthetische Polyelektrolyte wie die Amin- und Alkalisalze der Polyacrylsäure sowie anorganische Verdickungsmittel, beispielsweise kolloidales Magnesiumaluminiumsilikat oder disperses Siliciumoxid.

In den erfindungsgemäßen Zahnpasten können selbstverständlich auch weitere Wirkstoffe Verwendung finden. Solche sind insbesondere die bekannten kariesprophylaktischen Fluoride, vorzugsweise in einer solchen Menge daß die Konzentration an reinem Fluor im Mittel etwa 0,05 bis etwa 1 Gew.-%, vorzugsweise 0,1 bis 0,5 Gew.-% des Mittels beträgt.

Geeignete Fluorverbindungen sind insbesondere die verschiedenen Salze der Monofluorphosphorsäure wie Natrium-, Kalium-, Lithium-, Calcium- und Aluminiummono- und difluorphosphat sowie verschiedene, Fluor in ionisch gebundener Form enthaltene Fluoride, insbesondere Alkalifluoride wie Natrium-, Lithium-, Kalium- und Ammoniumfluorid, Zinnfluorid, Manganfluorid, Kupferfluorid, Zirkoniumfluorid und Aluminiumfluorid sowie Gemische oder Anlagerungsprodukte dieser Fluoride untereinander und mit anderen Fluorverbindungen, beispielsweise Alkalimanganfluoride.

Weitere, in den erfindungsgemäßen Zahnpasten einsetzbare Stoffe eine Mittel zur Verhinderung von Zahnbelagsbildung wie Chlorhexidinsalze, Zink- und Kupferverbindungen, Harnstoff, Hexetidin, Hesperidin, Allantoin, Azulen, weitere die Zahnsteinbildung verhindernde bzw. vermindernde Stoffe wie Alkylendiaminotetra-(methylenphosphonsäuren), etc..

Der pH-Wert der erfindungsgemäßen Zahnpasta liegt zwischen etwa 4 und etwa 10, vorzugsweise 5,5 und 9,0.

Eine Übersicht über in Zahnpasten einsetzbare Substanzen findet sich, wie überhaupt über sonstige zur Herstellung von Zahnpflegemitteln üblicherweise zum Einsatz gelangende Stoffe und die dabei angewandten Herstellungsverfahren, in dem Handbuch von M.S. Balsam und E. Sagarin, "Cosmetics - Science and Technology", 2nd Ed., Vol. 1, S. 423 bis 533 (1972), auf das hier ausdrücklich Bezug genommen wird.

Im folgenden werden einige Beispiele gegeben, die das Wesen der vorliegenden Erfindung charakterisieren:

Beispiel 1

α-Aluminiumoxidtrihydrat        38,00 (Gew.-%)
Sorbit        10,00 (Gew.-%)
Glycerin        5,00 (Gew.-%)
Methylcellulose        0,80 (Gew.-%)
Hydroxyethylcellulose        0,40 (Gew.-%)
Natriummonofluorphosphat        0,76 (Gew.-%)
Natriumfluorid        0,11 (Gew.-%)
Natriumlaurylsulfat        1,20 (Gew.-%)
Aromamischung        1,00 (Gew.-%)
Saccharin-Natrium        0,08 (Gew.-%)
Kolloidales Siliciumdioxid        0,50 (Gew.-%)
Methyl-p-hydroxybenzoat, Natriumsalz        0,25 (Gew.-%)
β-Pyridylmethanolhydrogentartrat        0,15 (Gew.-%)
EHDP, Trinatriumsalz        0,85 (Gew.-%)
Tetranatriumpyrophosphat        3,75 (Gew.-%)
Allantoin        0,25 (Gew.-%)
Wasser        ad 100,00 (Gew.-%)

Beispiel 2

Gefälltes Siliciumdioxid-Gel        23,50 (Gew.-%)
Siliciumdioxid-Aerogel        2,50 (Gew.-%)
Glycerin        9,00 (Gew.-%)

Sorbit    17,50 (Gew.-%)
Natriumlaurylsulfat    1,50 (Gew.-%)
Carboxymethylcellulose    1,25 (Gew.-%)
Natriumfluorid    0,25 (Gew.-%)
Aromamischung    1,10 (Gew.-%)
Saccharin-Natrium    0,05 (Gew.-%)
Kupfersulfat . $5H_2O$    0,25 (Gew.-%)
Hexetidin    0,05 (Gew.-%)
Methyl-p-hydroxybenzoat, Natriumsalz    0,20 (Gew.-%)
n-Propyl-p-hydroxybenzoat, Natriumsalz    0,10 (Gew.-%)
3-Pyridylmethanoltartrat    0,20 (Gew.-%)
Tetrakaliumpyrophosphat    3,10 (Gew.-%)
Wasser    ad 100,00 (Gew.-%)

## Beispiel 3

Unlösliches Natriummetaphosphat    38,00 (Gew.-%)
Sorbit    13,00 (Gew.-%)
Kolloidales Siliciumdioxid    2,80 (Gew.-%)
Hydroxyethylcellulose    1,80 (Gew.-%)
Methyl-p-hydroxybenzoat    0,10 (Gew.-%)
Benzoesäure    0,10 (Gew.-%)
Saccharin-Natrium    0,20 (Gew.-%)
Aromamischung    1,10 (Gew.-%)
Fettalkoholpolyglycolether    0,60 (Gew.-%)
1,2-Propandiol    4,00 (Gew.-%)
Titandioxid    0,80 (Gew.-%)
N,N',N'-Tri(2-hydroxyethyl)-N-octadecyl-1,3-diaminopropandihydrofluorid    2,00 (Gew.-%)
3-Pyridylmethanoldihydrogencitrat    0,25 (Gew.-%)
EHDP, Dinatriumsalz    1,60 (Gew.-%)
Wasser    ad 100,00 (Gew.-%)

## Beispiel 4

Polymethylmethacrylat-Pulver (mittl. Teilchengröße 1-5 μm)    15,00 (Gew.-%)
Hitzebehandeltes β-Calciumpyrophosphat    15,00 (Gew.-%)
Natriumcarboxymethylcellulose    1,20 (Gew.-%)
Natriumlauroylsarcosinat    0,80 (Gew.-%)
Natriumlaurylsulfat    0,80 (Gew.-%)
Natriummonofluorphosphat    1,20 (Gew.-%)
Saccharin-Natrium    0,10 (Gew.-%)
Aromamischung    1,10 (Gew.-%)
3-Pyridylmethanolgluconat    0,30 (Gew.-%)
Tetranatriumpyrophosphat    3,20 (Gew.-%)
EHDP, Dinatriumsalz    0,60 (Gew.-%)

Glycerin    10,00 (Gew.-%)
Sorbit    8,00 (Gew.-%)
Waser    ad 100,00 (Gew.-%)

## Beispiel 5

Silica-Xerogel    25,00 (Gew.-%)
Silica-Aerogel    2,80 (Gew.-%)
Sorbit    35,00 (Gew.-%)
Xanthum-Gum    1,10 (Gew.-%)
Natriumfluorid    0,30 (Gew.-%)
EHDP, Dinatriumsalz    0,80 (Gew.-%)
Dinatriumpyrophosphat    1,00 (Gew.-%)
3-Pyridylmethanoltartrat    0,10 (Gew.-%)
Tetranatriumpyrophosphat    1,80 (Gew.-%)
Natriumlaurylsulfat    1,30 (Gew.-%)
Natriumlauroylsarcosinat    0,80 (Gew.-%)
Aromamischung    1,00 (Gew.-%)
Saccharin-Natrium    0,10 (Gew.-%)
Methyl-p-hydroxybenzoat, Natriumsalz    0,30 (Gew.-%)
Farbstoff    q.s.
Wasser    ad 100,00 (Gew.-%)

## Beispiel 6

Calciniertes β-Calciumpyrophosphat    45,00 (Gew.-%)
Unlösliches Natriummetaphosphat    15,00 (Gew.-%)
Glycerin    5,50 (Gew.-%)
Sorbit    12,50 (Gew.-%)
Natriumcarboxymethylcellulose    1,20 (Gew.-%)
Natriumsulforicinoleat    0,80 (Gew.-%)
Natriumlaurylsulfat    1,20 (Gew.-%)
3-Pyridylmethanoltartrat    0,25 (Gew.-%)
Tetranatriumpyrophosphat    4,00 (Gew.-%)
Dinatriumpyrophosphat    1,20 (Gew.-%)
Natriummonofluorphosphat    1,14 (Gew.-%)
Saccharin-Natrium    0,11 (Gew.-%)
Aromagemisch    1,00 (Gew.-%)
Natriumbenzoat    0,30 (Gew.-%)
Methyl-p-hydroxybenzoat, Natriumsalz    0,15 (Gew.-%)
Wasser    ad 100,00 (Gew.-%)

**Ansprüche**

1. Zahnpasta, die im wesentlichen frei von wasserlösliche Calciumionen lieferndern Verbindungen ist, dadurch gekennzeichnet, daß sie eine Kombination aus

a) einem Di- und/oder Tetraalkalipyrophosphat in einer Menge von 1 bis 5 Gew.% (berechnet auf Pyrophosphat), bezogen auf die Gesamtzusammensetzung, und/oder Ethan-1-hydroxy-1,1-diphosphonsäure bzw. deren wasserlöslichen Salzen in einer Menge von 0,2 bis 2,0 Gew.% (berechnet auf freie Phosphonsäure), bezogen auf die Gesamtzusammensetzung und

b) einem wasserlöslichen Salz des 3-Pyridylmethanols in einer Menge von 0,05 bis 0,5% (berechnet auf 3-Pyridylmethanol), bezogen auf die Gesamtzusammensetzung, enthält.

2. Zahnpasta nach Anspruch 1, dadurch gekennzeichnet, daß sie das wasserlösliche Tartrat, Citrat, Gluconat, Lactat oder Aspartat des 3-Pyridylmethanols enthält.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | FR-A-2 220 246 (PROCTER & GAMBLE) <br> * Beispiele 2,14; Ansprüche 1,5,10,12 * <br> --- | 1-2 | A 61 K 7/16 |
| A | FR-A-2 318 632 (PIERRE FABRE) <br> * Das ganze Dokument * <br> --- | 1-2 | |
| A | G.A. NOWAK: "Die kosmetischen Präparate", Band 2, 3. Auflage,1984, Seiten 629-631, Verlag für chem. Industrie H. Ziolkowsky KG, Augsburg, DE <br> * Seite 629-630: "Wirkstoffe gegen Zahnstein"; Seiten 630-631: "Gingivitrope Wirkung" * <br> --- | 1-2 | |
| A | US-A-4 323 551 (J.J. PARRAN Jr.) <br> * Das ganze Dokument * <br> ----- | 1-2 | |

| | RECHERCHIERTE SACHGEBIETE (Int. Cl.4) |
|---|---|
| | A 61 K |

Der vorliegende Recherchenbericht wurde für alle Patentansprüche erstellt

| Recherchenort | Abschlußdatum der Recherche | Prüfer |
|---|---|---|
| DEN HAAG | 05-07-1988 | FISCHER J.P. |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus andern Gründen angeführtes Dokument

................................................................

& : Mitglied der gleichen Patentfamilie, übereinstimmendes
Dokument

EPO FORM 1503 03.82 (P0403)